# FASCICULE DE BREVET EUROPEEN

(11) **EP 0 722 304 B1**
(45) Date de publication et mention de la délivrance du brevet: **14.01.1998**
(21) Numéro de dépôt: 94929583.6
(22) Date de dépôt: 05.10.1994
(51) Int. Cl.: A61F 2/06

(54) **ORGANE TUBULAIRE EXPANSIBLE POUR ENDOPROTHESE INTRALUMINALE, ENDOPROTHESE INTRALUMINALE, PROCEDE DE FABRICATION**
TUBULARES DEHNBARES GLIED FÜR EINE INTRALUMINALE ENDOPROTHESE, INTRALUMINALE ENDOPROTHESE UND VERFAHREN ZUR HERSTELLUNG
TUBULAR EXPANDABLE MEMBER FOR AN INTRALUMINAL ENDOPROSTHESIS, INTRALUMINAL ENDOPROSTHESIS AND METHOD OF PRODUCTION

(30) Priorité: 05.10.1993 FR 9311854
(43) Date de publication de la demande: 24.07.1996
(73) Titulaire: ASSISTANCE PUBLIQUE, HOPITAUX DE PARIS, 75100 Paris (FR)
(72) Inventeur: BOUDGHENE, Frank, F-75019 Paris (FR); MICHEL, Jean-Baptiste, F-75012 Paris (FR); SAPOVAL, Marc, F-94110 Arcueil (FR); LAVASTE, François, F-91240 Saint-Michel-sur-Orge (FR)
(74) Mandataire: Le Guen, Gérard
(86) Numéro de dépôt international: FR9401164
(87) Numéro de publication internationale: WO9509584

(56) Documents cités:
- EP-A- 0 274 846
- EP-A- 0 335 341
- DE-U- 8 812 719
- US-A- 3 657 744

## Description

La présente invention est relative à un organe tubulaire expansible pour endoprothèse destinée à être implantée dans la lumière d'un vaisseau sanguin, notamment pour réparer des vaisseaux sanguins resserrés ou bouchés, tels que les artères coronaires, les artères périphériques ou les artères rénales, ou pour le traitement des anévrysmes, notamment de l'aorte chez l'être humain. Elle peut être également utilisée pour traiter des sténoses ou des fuites d'autres voies de passage que les vaisseaux dans le corps humain : voies biliaires, voies urinaires, voies aériennes, voies digestives ...

Il a été démontré par l'expérience qu'une prothèse endovasculaire intraluminale représente une possibilité de variante à la chirurgie vasculaire classique. La prothèse endovasculaire intraluminale implique l'insertion percutanée dans un vaisseau sanguin d'une greffe prothésique tubulaire, notamment métallique, et son amenée par l'intermédiaire d'un cathéter, vers l'emplacement désiré à l'intérieur du système vasculaire. Les avantages de ce procédé sur la chirurgie vasculaire classique sont que l'on évite la nécessité de pratiquer chirurgicalement l'exposition, l'incision, l'enlèvement, le remplacement ou la mise en dérivation du vaisseau sanguin défectueux.

Les endoprothèses pour le traitement des anévrysmes se composent outre de l'organe tubulaire qui a une fonction d'accrochage, d'un revêtement en tissu assurant l'étanchéité de la nouvelle aorte.

Les organes tubulaires métalliques d'accrochage (stents) sont de trois types : expansible, auto-expansible ou à mémoire thermique.

Les prothèses de type expansible sont les plus utilisées pour le traitement des sténoses. Elles comprennent un organe tubulaire métallique expansible sur lequel est fixé, dans le cas des endoprothèses destinées aux anévrysmes, le revêtement en tissu.

La prothèse est comprimée sur la sonde à ballonnet d'angioplastie et le tout est introduit dans une gaine. En retirant la gaine sur le lieu de largage, le ballonnet est gonflé et va créer une déformation plastique de la prothèse. Une fois implantée, le diamètre de la prothèse est celui qui a été donné, c'est-à-dire le diamètre de la sonde à ballonnet après gonflement.

Un des problèmes majeurs est lié à l'encombrement de la prothèse. En effet, dans le cas de l'aorte humaine par exemple, la prothèse une fois installée dans la lumière de l'aorte, doit avoir un diamètre de l'ordre de 20 mm mais ne doit à l'état initial pas avoir un diamètre de plus de 3 à 4 mm pour pouvoir être introduite dans le vaisseau et amenée au lieu de mise en place.

Il existe donc un besoin pour des organes tubulaires expansibles qui puissent atteindre un diamètre final de près de 20 mm tout en ayant un diamètre aussi petit que possible à l'état initial.

On connaît, notamment de EP 0364 787, un organe tubulaire expansible comportant des fentes formées dans la paroi de l'organe tubulaire disposées sensiblement parallèlement à l'axe longitudinal de l'organe tubulaire, chaque fente étant uniformément séparée selon un axe circonférentiel par rapport à la fente adjacente par des raccords transversaux, de façon à former des portions de tube longitudinales formées entre des fentes successives. Les extrémités de chaque fente sont disposées de manière intermédiaire par rapport à la fente adjacente de façon à ce que les raccords transversaux disposés à l'extrémité de chaque fente et entre les portions longitudinales soient à leur tour disposés de façon intermédiaire par rapport aux deux extrémités de la fente adjacente, les fentes adjacentes étant ainsi disposées en quinconce l'une par rapport à l'autre. Les portions longitudinales ont une largeur sensiblement identique à celle des raccords transversaux si bien que, lors de la mise en place de la prothèse, se produit une déformation des portions longitudinales essentiellement dans la partie située entre deux raccords transversaux successifs le long de l'axe longtudinal conférant à l'organe tubulaire des ouvertures en forme de losange à la fin de la dilatation.

Les inconvénients d'un tel organe tubulaire sont un manque de souplesse longitudinale, une diminution de la longueur finale après pose par rapport à la longueur initiale et des difficultés de sertissage sur le ballonnet de dilatation et de relargage.

La présente invention a pour but de fournir un organe tubulaire expansible pour endoprothèse intraluminale qui ne présente pas les inconvénients de l'état de la technique et qui soit le moins encombrant possible à l'état initial.

L'invention a donc pour objet un organe tubulaire pour endoprothèse intraluminale obtenu par découpe d'un tube et expansible par application d'une force, comportant des portions longitudinales raccordées deux à deux successivement par au moins une portion transversale, dans laquelle ladite portion transversale comporte au moins une ondulation déformable lors de l'expansion de l'organe.

Selon l'invention, l'organe tubulaire a une longueur constante déterminée par lesdites portions longitudinales et l'ondulation déformable est prévue de sorte que la déformation des portions transversales a un effet exclusivement sur la dimension radiale de l'organe tubulaire.

Avantageusement, l'organe tubulaire comporte une portion transversale de raccordement à chaque extrémité des portions longitudinales. Les portions transversales de raccordement sont de préférence disposées symétriquement. Selon un mode de réalisation préféré, les portions transversales de raccordement sont disposées en regard l'une de l'autre.

En variante, l'organe tubulaire selon l'invention comporte au moins une portion transversale de raccordement disposée dans la partie intermédiaire des portions longitudinales.

Avantageusement, les ondulations se présentent sous la forme générale d'un U dont les extrémités des branches latérales sont reliées aux portions longitudinales de l'organe.

De manière avantageuse, les portions longitudinales et les portions transversales présentent la même épaisseur et les portions longitudinales ont une largeur supérieure à la largeur des portions transversales, de préférence égale au moins au double de la largeur des portions transversales.

Selon un autre mode de réalisation de l'invention, les portions longitudinales successives comportent des parties en saillie de renfort en regard l'une de l'autre.

L'organe tubulaire est constitué d'un métal, de préférence de l'acier inoxydable.

L'organe tubulaire selon l'invention peut être utilisé tel quel pour former une prothèse intraluminale pour le traitement de sténoses par dilatation intraluminale.

Il peut également être utilisé pour le traitement d'anévrysmes par une endoprothèse intraluminale expansible, où il formera la partie centrale, une gaine périphérique en un matériau étanche étant alors fixée à l'organe tubulaire, la gaine étant avantageusement formée de fibres polyester tissés, de préférence en Dacron®.

L'invention a également pour objet un procédé de fabrication d'un organe tubulaire comme décrit ci-dessus, caractérisé en ce que l'on découpe au laser des portions longitudinales et des portions transversales à partir d'un tube creux ayant un diamètre et une épaisseur de paroi identique au diamètre et à l'épaisseur de paroi de l'organe tubulaire final.

L'invention sera décrite en détail ci-après en se référant aux Figures annexées dans lesquelles :
- la Fig. 1 représente une vue en perspective avec arrachement partiel, d'une endoprothèse vasculaire selon l'invention comprenant un premier mode de réalisation d'un organe tubulaire expansible et une gaine périphérique ;
- la Fig. 2 représente une vue plane du développé de l'organe tubulaire expansible représenté à la Fig. 1 ;
- la Fig. 3 représente une vue plane du cadre II de la Fig. 2 après expansion de l'organe tubulaire par dilatation ;
- la Fig. 4 représente une vue plane du développé de l'organe tubulaire selon un second mode de réalisation ;
- la Fig. 5 représente une vue plane du développé de l'organe tubulaire selon un troisième mode de réalisation.

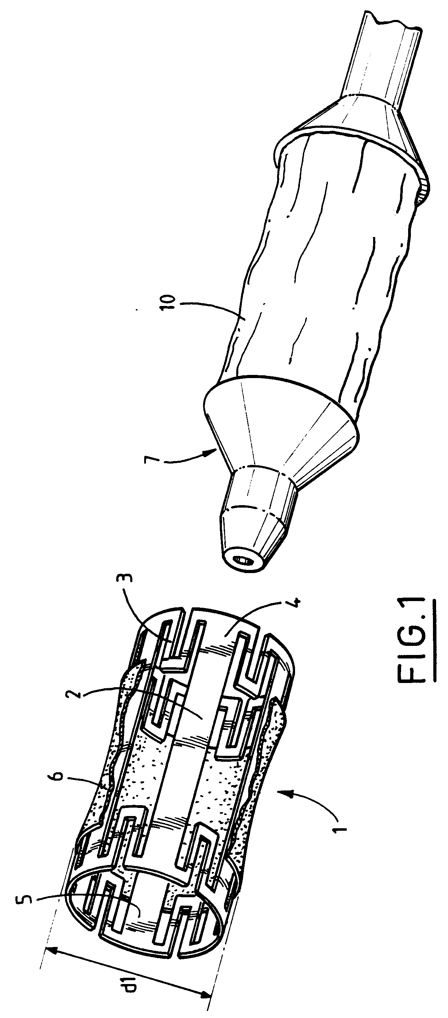

La figure 1 représente une endoprothèse vasculaire du type destinée à traiter les anévrysmes, à l'état initial, comprenant un organe tubulaire 1 dont la paroi comprend des parties longitudinales 2 et des portions transversales 3 en forme de U servant d'éléments de raccord entre deux portions longitudinales successives, disposées aux extrémités 4 et 5 de chaque portion longitudinale 2.

Sur cet organe tubulaire 1 est fixée une gaine 6 en polyester tissé. La gaine 6 en polyester tissé est fixée à l'organe tubulaire 1 par couture à points séparés entre les éléments métalliques de l'organe tubulaire et le tissu de la gaine 6. La gaine 6 a une longueur inférieure à l'organe tubulaire 1, de manière à ce que l'organe tubulaire l dépasse d'environ 10 mm à chaque extrémité comme représenté à la figure 1.

L'endoprothèse vasculaire représentée sur la figure 1 est sertie sur un cathéter 7 à ballonnet d'angioplastie 10 représenté ici à l'état non gonflé.

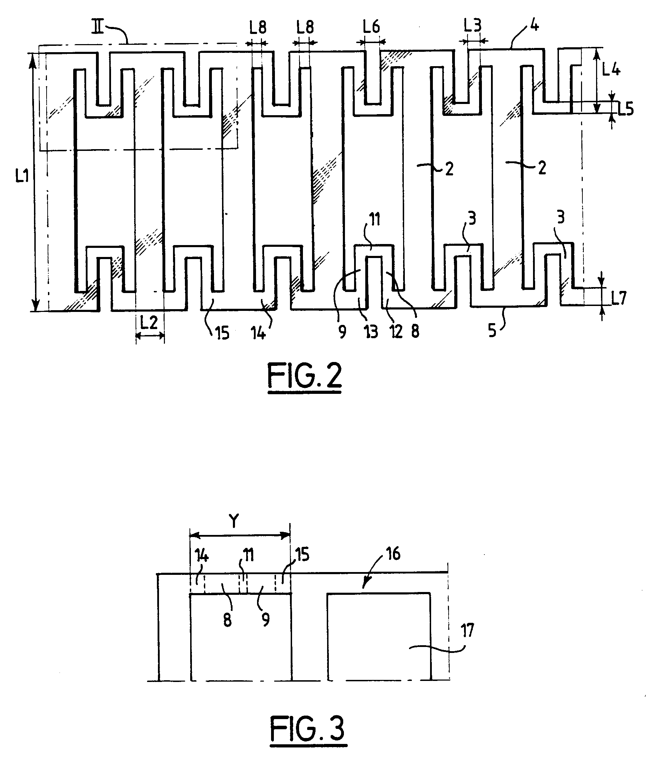

Comme représenté sur la figure 2, l'organe tubulaire comporte six portions longitudinales 2 parallèles orientées suivant l'axe longitudinal de l'organe tubulaire.

Chaque portion longitudinale 2 est reliée par ses extrémités 4 et 5 à la portion longitudinale 2 adjacente par une portion transversale 3 ayant la forme d'un U comprenant des branches latérales 8 et 9 reliées entre elles par une branche intermédiaire 11. Les extrémités 12 et 13 des branches latérales de la portion transversale 3 en forme de U sont reliées aux extrémités 4 et 5 de portions longitudinales 2 au moyen des segments de liaison 14 et 15.

L'organe tubulaire représenté à la figure 1 a les dimensions suivantes :
- diamètre (d₁) de l'élément tubulaire : 3 mm
- épaisseur de la paroi de l'élément tubulaire : 0,3 mm
- L₁ : longueur de l'organe tubulaire : 60 mm
- L₂ : largeur des portions longitudinales 2 : 0,79 mm
- L₃ : largeur des branches latérales 8 et 9 de la portion transversale 3 en forme de U : 0,2 mm
- L₄ : longueur des branches latérales 8 et 9 de la portion transversale 3 en forme de U : 4,7 mm
- L₅ : longueur de la branche intermédiaire 11 : 0,25 mm
- L₆ : largeur de la branche intermédiaire 11 : 0,125 mm
- L₇ : longueur des segments de liaison 14 et 15 : 0,25 mm
- L₈ : largeur des segments de liaison 14 et 15 : 0,125 mm.

La construction décrite ci-dessus de la prothèse permet à cette dernière d'être expansée de façon uniforme et vers l'extérieur, d'une manière contrôlée pour passer à la configuration représentée sur la figure 1 ou 2, lors de l'application d'une force adéquate, depuis l'intérieur de l'organe tubulaire 1, comme décrit ci-dessous avec plus de détails.

De façon classique, le cathéter 7 et la prothèse formée par l'organe tubulaire expansible 1 et la gaine de polyester 6 sont amenés à l'emplacement souhaité, pour expanser la lumière de la voie de passage dans le corps là où l'on souhaite implanter la prothèse. La fluoroscopie, l'échographie ou d'autres techniques classiques peuvent être utilisées pour s'assurer que le cathéter et la prothèse sont amenés à l'emplacement souhaité, à l'intérieur de la voie de passage dans le corps. La prothèse est ensuite expansée et déformée de façon contrôlée, en dilatant de façon contrôlée le ballon d'angioplastie 10 du cathéter 7, de manière que la prothèse soit expansée et déformée radialement, vers l'extérieur, pour venir en contact avec la voie de passage dans le corps. Après avoir accompli l'expansion et la déformation souhaitées de la prothèse, le ballon d'angioplastie peut être plié ou dégonflé et le cathéter 7 peut être retiré de façon classique de la voie de passage dans le corps. Si on le souhaite, le cathéter 7 et la prothèse peuvent être initialement entourés par une gaine en Teflon classique, qui est retirée de la prothèse avant l'expansion de cette dernière.

L'organe tubulaire de la prothèse présente initialement le premier diamètre d₁ plié et prédéterminé, comme décrit en liaison avec les figures 1 et 2, afin de permettre l'insertion de l'organe tubulaire 1 dans la voie de passage dans le corps, comme décrit ci-dessus. Lorsque l'on souhaite implanter la prothèse à l'intérieur d'une voie de passage dans le corps, la prothèse est expansée et déformée de façon contrôlée vers un second diamètre d₂, ce dernier étant variable et déterminé par le diamètre intérieur de la voie de passage dans le corps et par l'ampleur de l'expansion du ballon d'angioplastie gonflable 10 du cathéter 7. De façon correspondante, la prothèse expansée et déformée, lors du gonflement du ballon d'angioplastie 10, ne va pas pouvoir migrer de l'emplacement souhaité, à l'intérieur de la voie de passage dans le corps, et l'expansion de la prothèse ne va pas pouvoir non plus, de façon analogue, provoquer de rupture de la voie de passage dans le corps. En outre, dans la mesure où la prothèse n'est pas "élastique" ni un "organe auto-expansible", la prothèse n'applique pas de façon continue une force radiale dirigée vers l'extérieur, contre la surface intérieure de la voie de passage dans le corps qui dépasse celle nécessaire pour résister à un pliage radial de la voie de passage dans le corps. Ainsi, l'érosion de la surface intérieure ou de l'intima, de l'artère ou voie de passage dans le corps, est empêchée.

Lorsque l'on souhaite utiliser l'organe tubulaire expansible intraluminal pour dilater la lumière d'une voie de passage dans le corps présentant une aire de sténose, l'expansion de l'organe tubulaire intraluminal 1 à l'aide du ballon d'angioplastie 10 permet une dilatation contrôlée de l'aire sténotique et en même temps, une expansion et une déformation contrôlées de l'endoprothèse vasculaire formée par l'organe tubulaire expansible 1, de sorte que cette dernière empêche la voie de passage dans le corps de se plier et de diminuer la taille de la lumière expansée au préalable. De nouveau, le second diamètre expansé de l'endoprothèse intraluminale est variable et déterminé par le diamètre intérieur expansé souhaité de la voie de passage dans le corps. Ainsi, l'endoprothèse expansible intraluminale ne va pas migrer de l'emplacement souhaité à l'intérieur de la voie de passage dans le corps lors du dégonflement du ballon d'angioplastie et l'expansion de l'endoprothèse intraluminale ne vas pas non plus, de façon analogue, provoquer de rupture de la voie de passage dans le corps ni d'érosion. En outre, si un volet intimal ou fissure se formait dans la voie de passage dans le corps, à l'endroit de l'endoprothèse, cette dernière va assurer que ce volet intimal ne puisse pas se plier vers l'intérieur, dans la voie de passage dans le corps, ni se détacher par déchirure et circuler à travers la voie de passage dans le corps. Dans le cas d'utilisation de l'endoprothèse, pour expanser la lumière d'une partie de voie de passage critique dans le corps, telle que l'artère coronaire principale gauche, le volet intimal ne va pas pouvoir boucher l'artère coronaire principale gauche du coeur ni provoquer la mort du patient.

La figure 3 représente l'élément tubulaire 1 de la Fig. 2 à l'état expansé théorique après dilatation par le ballonnet d'angioplastie 10 comme représenté sur la Fig. 1. Les branches latérales 8,9 et la branche intermédiaire 11 du U complètement dépliées forment avec les segments de liaison 14 et 15 une portion rectiligne 16 de longueur Y reliant chaque élément longitudinal successif 2, de sorte que l'élément tubulaire 1 se présente sous la forme générale d'un tube dont la paroi est formée de portions longitudinales 2 et de fentes 17 de forme sensiblement rectangulaire.

Après expansion les dimensions de l'élément tubulaire sont :
d₂ : diamètre de l'élément tubulaire : ≃ 20 mm
L₂ : longueur de l'élément tubulaire : 60 mm
Y ≃ 9,21 mm.

En pratique, l'élément tubulaire peut ne pas être complètement expansé. Des variations d'expansion sont même souhaitables (dans une plage restreinte) pour que les dimensions finales du tube après dilatation correspondent exactement aux dimensions du vaisseau où il est implanté (variant pour chaque patient).

L'organe tubulaire 1 peut être un matériau adéquat quelconque qui est compatible avec le corps humain et les fluides corporels, avec lesquels la prothèse vasculaire peut entrer en contact.

L'organe tubulaire 1 doit également être réalisé en un matériau qui présente les caractéristiques de résistance et d'élasticité nécessaires pour permettre à l'organe tubulaire 1 d'être expansé et déformé de la configuration représentée à la figure 2 pour passer à la configuration représentée à la figure 3 et en outre pour permettre à l'organe tubulaire 1 de maintenir sa configuration expansée et déformée avec le diamètre augmenté d₂ correspondant à l'état représenté sur la figure 3 et de résister à un pliage radial. Des matériaux adéquats pour la fabrication de l'organe tubulaire 1 comprennent l'argent, le tantale, l'acier inoxydable, l'or, le titane ou toute matière synthétique adéquate présentant les caractéristiques requises décrites précédemment.

De préférence, l'organe tubulaire 1 est initialement un tube en acier inoxydable, à paroi mince, présentant une épaisseur de paroi uniforme, un matériau particulièrement préféré étant un acier inox 316L.

L'organe tubulaire 1 représenté à la Fig. 2 a été obtenu par découpe d'un tube en inox ayant un diamètre de 3 mm, et une épaisseur de paroi de 0,3 mm au laser YAG, les paramètres étant les suivants :
Fréquence = 196 Hz
Largeur d'impulsion = 0,6 MS Energie = 0,4 Joules
Courant = 3 KV
Vitesse linéaire = 0,3 mm/mn
Focale = 80 mm
Télescope - diamètre du faisceau = 1/10 mm
Gaz - oxygène à 3 bars.

L'appareil utilisé était équipé d'une table "micro contrôle" de dimensions :
en X = 250 mm
en Y = 200 mm.

La table était asservie par des moteurs pas à pas d'incrément 5 µm.

Pour la réalisation des pièces, on a utilisé un plateau tournant muni d'une contre pointe.

Le contrôle de la découpe était réalisé visuellement à la loupe.

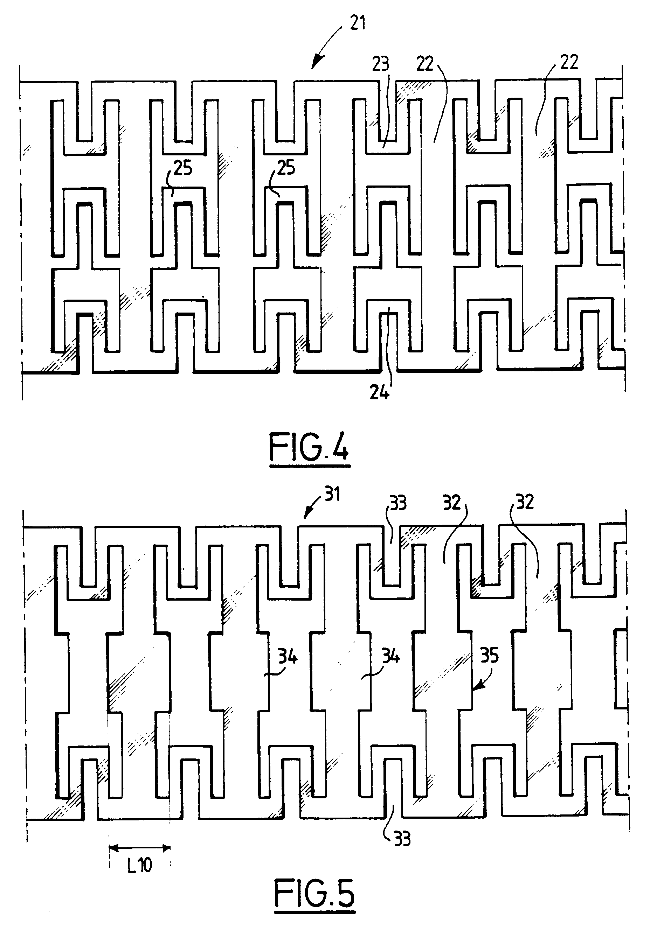

La figure 4 représente la découpe d'un second mode de réalisation de l'organe tubulaire selon l'invention.

Dans ce mode de réalisation, le dispositif tubulaire 21 comprend des portions longitudinales 22 au nombre de six et des portions transversales 23 et 24 au nombre de 12 disposées aux extrémités de l'organe tubulaire et reliant deux à deux les portions longitudinales successives ayant une forme et une disposition sensiblement identiques à celles de l'organe tubulaire 1 décrit aux figures 1 à 3, les portions transversales 23 étant disposées à l'une des extrémités de l'organe tubulaire 21 et les portions transversales 24 à l'autre extrémité.

Dans ce mode de réalisation, l'organe tubulaire 21 comprend également des portions transversales de raccordement 25 disposées dans la partie intermédiaire des portions longitudinales 22, de manière symétrique et en direction des portions transversales 23 de l'une des extrémités et en direction opposée des portions transversales 24 de l'autre extrémité de l'organe tubulaire 21.

Les dimensions des différents éléments sont sensiblement les mêmes que les dimensions indiquées pour les différents éléments de l'organe tubulaire représenté aux figures 1 à 3.

Dans une variante de ce mode de réalisation non représentée, l'organe tubulaire 21 comprend 8 portions longitudinales au lieu de 6 reliées deux à deux à chaque extrémité par des portions transversales 23 et 24 et dans leur partie intermédiaire par des portions transversales 25 dont la configuration et les dimensions sont sensiblement les mêmes que celles des portions transversales représentées à la figure 4. Les portions longitudinales ont dans ce cas une largeur d'environ 0,4 mm.

Dans le mode de réalisation représenté à la figure 5, l'organe tubulaire 31 comprend des portions longitudinales 32 et des portions transversales 33 disposées à chaque extrémité des portions longitudinales 32 et raccordant chaque portion longitudinale 32 à la portion longitudinale 32 directement adjacente, ayant une disposition et des dimensions sensiblement identiques à celles des portions transversales représentées aux figures 2 et 3.

Les portions longitudinales successives 32 comportent des parties en saillie de renfort 34 disposées en regard l'une de l'autre dans la partie intermédiaire 35 des portions longitudinales 32. La partie intermédiaire de chaque portion longitudinale a dans ce mode de réalisation une largeur L₁₀ de 1,2 mm, les dimensions des autres éléments y compris des extrémités des parties longitudinales 32 étant par ailleurs sensiblement identiques à celles de l'organe tubulaire représenté à la figure 2.

Les dimensions indiquées pour les figures 1 à 4 sont des dimensions adaptées à l'utilisation de l'endoprothèse intraluminale pour le traitement des anévrysmes aortiques.

Il va de soi que les dimensions peuvent être aisément déterminées par l'homme du métier à partir de la description ci-dessus pour d'adaptation d'une endoprothèse selon l'invention au traitement des anévrysmes ou au traitement de sténoses d'autres vaisseaux ou voies de passages de diamètre moindre.

Enfin, la partie tissulaire ou synthétique assurant l'étanchéité peut être attachée à une hauteur variable sur la partie métallique.

## Revendications

1. Organe tubulaire (1) pour endoprothèse intraluminale obtenu par découpe d'un tube et expansible par application d'une force comportant des portions longitudinales (2, 22, 32) raccordées deux à deux successivement par au moins une portion transversale (3, 23, 33), caractérisé en ce que ladite portion transversale comporte au moins une ondulation déformable lors de l'expansion de l'organe, cette ondulation déformable étant prévue de sorte que la déformation des portions transversales a un effet exclusivement sur la dimension radiale de l'organe tubulaire.

2. Organe tubulaire selon la revendication 1, caractérisé en ce qu'il comporte une portion transversale (3, 23, 33) de raccordement à chaque extrémité des portions longitudinales.

3. Organe tubulaire selon l'une des revendications précédentes, caractérisé en ce qu'il comporte des portions transversales (3, 23, 33) de raccordement disposées symétriquement.

4. Organe tubulaire selon l'une des revendications précédentes, caractérisé en ce qu'il comporte des portions transversales (3, 23, 33) de raccordement disposées en regard l'une de l'autre.

5. Organe tubulaire selon l'une des revendications précédentes, caractérisé en ce qu'il comporte au moins une portion transversale (3, 23, 33) de raccordement disposée dans la partie intermédiaire des portions longitudinales.

6. Organe tubulaire selon l'une des revendications précédentes, caractérisé en ce que les ondulations se présentent sous la forme générale d'un U dont les extrémités des branches latérales (8 et 9) sont reliées aux portions longitudinales (2, 22, 32) de l'organe.

7. Organe tubulaire selon l'une des revendications précédentes, caractérisé en ce que les portions longitudinales (2, 22, 32) et les portions transversales (3, 23, 33) présentent la même épaisseur et en ce que les portions longitudinales (2, 22, 32) ont une largeur supérieure à la largeur des portions transversales (3, 23, 33).

8. Organe tubulaire selon la revendication 8, caractérisé en ce que les portions longitudinales (2, 22, 32) ont une largeur sensiblement égale à au moins le double de la largeur des portions transversales (3, 23, 33).

9. Organe tubulaire selon l'une quelconque des revendications précédentes, caractérisé en ce que les portions longitudinales (32) successives comportent des parties en saillie (34) de renfort en regard l'une de l'autre.

10. Organe tubulaire selon l'une quelconque des revendications précédentes, caractérisé en ce qu'il est en acier inoxydable.

11. Endoprothèse intraluminale expansible pour le traitement de sténoses, caractérisée en ce qu'elle comprend un organe tubulaire (1) selon l'une quelconque des revendications précédentes.

12. Endoprothèse vasculaire intraluminale notamment pour le traitement d'anévrysmes, caractérisée en ce qu'elle comprend une partie centrale formée d'un organe tubulaire expansible selon l'une quelconque des revendications 1 à 10 et une partie périphérique formée d'une gaine (6).

13. Endoprothèse intraluminale selon la revendication 12, caractérisée en ce que la gaine est formée de fibres polyester tissées.

14. Procédé pour la fabrication d'un organe tubulaire selon l'une quelconque des revendications 1 à 10, caractérisé en ce que l'on découpe au laser des portions longitudinales (2, 22, 32) et des portions transversales (3, 23, 33) à partir d'un tube creux.

## Claims

1. A tubular member (1) for an intraluminal endoprosthesis, obtained by cutting up a tube and expansible through application of a force, this tubular member (1) comprising longitudinal portions (2, 22, 32) successively connected in pairs by at least one transverse portion (3, 23, 33), characterised in that the said transverse portion comprises at least one undulation deformable upon expansion of the member, this deformable undulation being provided such that deformation of the transverse portions has an effect solely on the radial dimension of the tubular member.

2. A tubular member in accordance with Claim 1, characterised in that it comprises a transverse connecting portion (3, 23, 33) at each end of the longitudinal portions.

3. A tubular member in accordance with one of the preceding Claims, characterised in that it comprises symmetrically-arranged transverse connecting portions (3, 23, 33).

4. A tubular member in accordance with one of the preceding Claims, characterised in that it comprises transverse connecting portions (3, 23, 33) arranged facing one another.

5. A tubular member in accordance with one of the preceding Claims, characterised in that it comprises at least one transverse connecting portion (3, 23, 33) arranged in the intermediate part of the longitudinal portions.

6. A tubular member in accordance with one of the preceding Claims, characterised in that the undulations are in the general form of a U of which the ends of the lateral arms (8 and 9) are joined to the longitudinal portions (2, 22, 32) of the member.

7. A tubular member in accordance with one of the preceding Claims, characterised in that the longitudinal portions (2, 22, 32) and the transverse portions (3, 23, 33) are the same thickness and in that the longitudinal portions (2, 22, 32) are wider than the transverse portions (3, 23, 33).

8. A tubular member in accordance with Claim 8[sic], characterised in that the width of the longitudinal portions (2, 22, 32) is substantially equal to at least double the width of the transverse portions (3, 23, 33).

9. A tubular member in accordance with any one of the preceding Claims, characterised in that the successive longitudinal portions (32) comprise strengthening projections (34) facing one another.

10. A tubular member in accordance with any one of the preceding Claims, characterised in that it is made of stainless steel.

11. An expansible intraluminal endoprosthesis for treating stenoses, characterised in that it comprises a tubular member (1) in accordance with any one of the preceding Claims.

12. An intraluminal vascular endoprosthesis notably for treating aneurysms, characterised in that it comprises a central part consisting of an expansible tubular member in accordance with any one of Claims 1 to 10 and a peripheral part consisting of a sheath (6).

13. An intraluminal endoprosthesis in accordance with Claim 12, characterised in that the sheath consists of woven polyester fibres.

14. A method of manufacturing a tubular member in accordance with any one of Claims 1 to 10, characterised in that longitudinal portions (2, 22, 32) and transverse portions (3, 23, 33) are cut from a hollow tube by means of a laser.

## Patentansprüche

1. Röhrenförmiges Element (1) für intraluminale Endoprothese, das durch Zerschneiden eines Rohrs erhalten wird und durch Ausübung einer Kraft ausgedehnt werden kann, enthaltend longitudinale Abschnitte (2, 22, 32), die paarweise nacheinander mit wenigstens einem transversalen Abschnitt (3, 23, 33) verbunden sind, dadurch gekennzeichnet, daß der transversale Abschnitt wenigstens eine bei der Ausdehnung des Elements verformbare Welligkeit aufweist, wobei diese verformbare Welligkeit vorgesehen ist, damit sich die Verformung der transversalen Abschnitte ausschließlich auf die radiale Dimension des röhrenförmigen Elements auswirkt.

2. Röhrenförmiges Element nach Anspruch 1, dadurch gekennzeichnet, daß es einen transversalen Abschnitt (3, 23, 33) für die Verbindung mit jedem Ende der longitudinalen Abschnitte enthält.

3. Röhrenförmiges Element nach einem der vorangehenden Ansprüche, dadurch gekennzeichnet, daß es transversale Verbindungsabschnitte (3, 23, 33) enthält, die symmetrisch angeordnet sind.

4. Röhrenförmiges Element nach einem der vorangehenden Ansprüche, dadurch gekennzeichnet, daß es transversale Verbindungsabschnitte (3, 23, 33) enthält, die einander gegenüber angeordnet sind.

5. Röhrenförmiges Element nach einem der vorangehenden Ansprüche, dadurch gekennzeichnet, daß es wenigstens einen transversalen Verbindungsabschnitt (3, 23, 33) enthält, der im Zwischenteil der longitudinalen Abschnitte angeordnet ist.

6. Röhrenförmiges Element nach einem der vorangehenden Ansprüche, dadurch gekennzeichnet, daß die Welligkeiten die allgemeine Form eines U besitzen, wobei die Enden der seitlichen Schenkel (8 und 9) mit den longitudinalen Abschnitten (2, 22, 32) des Elements verbunden sind.

7. Röhrenförmiges Element nach einem der vorangehenden Ansprüche, dadurch gekennzeichnet, daß die longitudinalen Abschnitte (2, 22, 32) und die transversalen Abschnitte (3, 23, 33) die gleiche Dicke besitzen und daß die longitudinalen Abschnitte (2, 22, 32) eine Breite besitzen, die größer als die Breite der transversalen Abschnitte (3, 23, 33) ist.

8. Röhrenförmiges Element nach Anspruch 8, dadurch gekennzeichnet, daß die longitudinalen Abschnitte (2, 22, 32) eine Breite besitzen, die im wesentlichen wenigstens gleich der doppelten Breite der transversalen Abschnitte (3, 23, 33) ist.

9. Röhrenförmiges Element nach irgendeinem der vorangehenden Ansprüche, dadurch gekennzeichnet, daß die aufeinanderfolgenden longitudinalen Abschnitte (32) vorspringende Verstärkungsteile (34), die einander gegenüberliegen, enthalten.

10. Röhrenförmiges Element nach irgendeinem der vorangehenden Ansprüche, dadurch gekennzeichnet, daß es aus rostfreiem Stahl ist.

11. Ausdehnbare intraluminale Endoprothese für die Behandlung von Stenosen, dadurch gekennzeichnet, daß sie ein röhrenförmiges Element (1) nach irgendeinem der vorangehenden Ansprüche enthält.

12. Intraluminale Gefäß-Endoprothese, insbesondere für die Behandlung von Aneurysmen, dadurch gekennzeichnet, daß sie einen mittleren Teil, der aus einem ausdehnbaren röhrenförmigen Element nach irgendeinem der Ansprüche 1 bis 10 sowie einen Umfangsteil, der aus einer Hülse (6) gebildet ist, enthält.

13. Intraluminale Endoprothese nach Anspruch 12, dadurch gekennzeichnet, daß die Hülse aus gewebten Polyesterfasern gebildet ist.

14. Verfahren zum Herstellen eines röhrenförmigen Elements nach irgendeinem der Ansprüche 1 bis 10, dadurch gekennzeichnet, daß ausgehend von einem Hohlrohr die longitudinalen Abschnitte (2, 22, 32) und die transversalen Abschnitte (3, 23, 33) mittels eines Lasers zerschnitten werden.
